# EUROPEAN PATENT SPECIFICATION

(11) **EP 2 839 280 B1**
(45) Date of publication and mention of the grant of the patent: **28.12.2016**
(21) Application number: 13777793.4
(22) Date of filing: 12.04.2013
(51) Int. Cl.: G01N 33/50

(54) **DEVICE FOR PERFORMING AN ENZYME-BASED DIAGNOSTIC TEST AND METHODS FOR USE THEREOF**
VORRICHTUNG ZUR DURCHFÜHRUNG EINES DIAGNOSTISCHEN TESTS AUF ENZYMBASIS UND VERFAHREN ZUR VERWENDUNG DAVON
DISPOSITIF POUR EFFECTUER UN TEST DIAGNOSTIQUE À BASE D'ENZYME ET PROCÉDÉS POUR L'UTILISATION DE CELUI-CI

(30) Priority: 17.04.2012 US 201261625390 P; 21.12.2012 US 201261740975 P
(43) Date of publication of application: 25.02.2015
(73) Proprietor: Ehrenkranz, Joel R.L., Salt Lake City, UT 84103 (US)
(72) Inventor: Ehrenkranz, Joel R.L., Salt Lake City, UT 84103 (US)
(74) Representative: Elsy, David
(86) International application number: PCT/US2013/036453
(87) International publication number: WO 2013/158505

(56) References cited:
- WO-A1-2009/063185
- US-A- 5 804 452
- US-A- 5 914 245
- US-A1- 2003 091 975
- US-A1- 2007 287 186
- US-A1- 2010 285 490
- US-A1- 2011 236 879
- NICOLA LEE DELL ET AL: "Towards a point-of-care diagnostic system", PROCEEDINGS OF THE 5TH ACM WORKSHOP ON NETWORKED SYSTEMS FOR DEVELOPING REGIONS, NSDR '11, 28 June 2011 (2011-06-28), page 3, XP055203646, New York, New York, USA DOI: 10.1145/1999927.1999931 ISBN: 978-1-45-030739-0
- ZACHARY J. SMITH ET AL: "Cell-Phone-Based Platform for Biomedical Device Development and Education Applications", PLOS ONE, vol. 6, no. 3, 2 March 2011 (2011-03-02), page e17150, XP055203456, DOI: 10.1371/journal.pone.0017150

## Description

### RELATED APPLICATIONS

This application claims the benefit of and priority to U.S. Prov. Pat. App. Ser. No. 61/625,390 filed 17 April 2012 and U.S. Prov. Pat. App. Ser. No. 61/740,975 filed 21 December 2012.

### BACKGROUND

Sampling and testing of biological samples and body fluids (e.g., saliva, blood, urine, fecal matter, foods, plants, fish, minerals, animals, etc) is common for both testing and monitoring humans, fish, animals, and plants for any number of biochemical or physiological conditions and, of course, for determining the general state of health of an organism. For example, sampling and testing of human body fluids is often performed for point-of-care testing ("POCT"). POCT is defined as medical testing at or near the site of patient care. The driving notion behind POCT is to bring the test conveniently and immediately to the patient. This increases the likelihood that the patient, physician, and care team will receive the results more quickly. This allows for immediate clinical management decisions to be made. POCT examples include, but are not limited to, blood glucose testing, metabolic testing (e.g., thyroid stimulating hormone), blood gas and electrolytes analysis, rapid coagulation testing, rapid cardiac markers diagnostics, drugs of abuse screening, urine testing, pregnancy testing, fecal occult blood analysis, food pathogen screening, hemoglobin diagnostics, infectious disease testing, cholesterol screening, cancer testing (e.g. PSA), hormone testing (hCG, LH, FSH), cardiac (troponin), pulmonary, gastroenterology (e.g., H. pylori antibodies), urology, nephrology, dermatology, neurology, pediatrics, surgical, and public health (Ebola, cholera, HIV, malaria), and combinations thereof.

One testing method that is often employed for POCT and more conventional testing involves the use of lateral-flow chromatographic immunoassay cassettes. Lateral-flow chromatographic immunoassay cassettes can be used to easily and quickly obtain a variety of qualitative results relating to a number of biochemical and physiological conditions and disease states of an individual. These kinds of tests require the end user to simply add a sample to the cassette and then observe the result a few minutes later. Since such rapid and easy-to-use tests are user friendly, they are very popular in both the professional and consumer markets nowadays. Such tests are also very popular in areas where access to trained health care professionals is limited or where access to proper medical facilities is limited (e.g., poor areas, developing countries, war zones, etc).

Lateral flow chromatographic immunoassay methods and devices have been described extensively. See, e.g., Gordon and Pugh, U.S. Pat. No. 4,956,302; H. Buck, et al., WO 90/06511; T. Wang, U.S. Pat. No. 6,764,825; W. Brown, et al., U.S. Pat. No. 5,008,080; Kuo and Meritt, US 6,183,972, EP 00987551A3. Such assays involve the detection and determination of an analyte substance that is a member of a specific binding pair consisting of a ligand and a receptor. The ligand and the receptor are related in that the receptor specifically binds to the ligand, being capable of distinguishing a specific ligand or ligands from other sample constituents having similar characteristics. Immunological assays involving reactions between antibodies and antigens are one such example of a specific binding assay. Other examples include DNA and RNA hybridization reactions and binding reactions involving hormones and other biological receptors. One well-known commercial embodiment of this technique is the Clearblue One-Step Pregnancy Test.

Lateral flow chromatographic immunoassay test cassettes have a number of desirable characteristics including their ease of use and broad applicability to a variety of analytes. Likewise, immunoassay procedures capable of being carried out on a test strip and which can be administered in the field or other locations where medical testing laboratories are not readily available have provided a great benefit to the diagnosis and control of disease. Currently, however, such lateral flow chromatographic immunoassay tests are generally only capable of providing qualitative results. That is, while currently available lateral flow chromatographic immunoassay test cassettes and cassette reader apparatuses are particularly well-suited for telling a practitioner whether or not one or more test substances are present in a sample above a given detection limit, they are poorly suited for providing quantitative results. There is an ongoing need in the art for devices and methods that combine the ease of use characteristics of lateral flow chromatographic immunoassay tests with systems that are designed to provide quantitative results. Such devices and methods may, for example, allow medical practitioners to diagnose a variety of conditions at the point of care (e.g., chair-side or essentially anywhere in the world) without being tied to a medical facility or a testing laboratory.

WO2009063185 discloses a method of reading, recording and optionally transmitting results which may be quantitative using any commercially available pocket PDA or computer.

### BRIEF SUMMARY

Disclosed herein is a an enzyme-based assay system for quantification of at least one of the activity level or the concentration of at least one enzyme or an enzyme substrate in a biological sample, the enzyme-based assay system comprising; a lateral-flow chromatographic assay cassette having an enzymatically activated detectable label configured for assaying a reaction involving an enzyme and a substrate, the lateral-flow chromatographic assay cassette including a sample application zone in fluid communication with a test zone via a fluid transport matrix, wherein the enzymatically activated detectable label is immobilized in the test zone, and wherein the analyte is one of an enzyme or an enzyme substrate; a testing device for data collection and data analysis, the testing device comprising a handheld computing device selected from the group consisting of a handheld digital camera device, a cellular phone, a smart phone, and a tablet computer, the testing device including; a testing apparatus configured to be coupled to the handheld computing device and configured to position the lateral-flow chromatographic assay cassetten proximity to a detector and a light source to control for focal length from the detector to the lateral-flow chromatographic assay cassette and and to control for illumination of the lateral-flow chromatographic assay cassette by the light source; the light source being capable of transmitting at least one wavelength of light configured to yield a detectable signal from the enzymatically activated detectable label; and the detector comprising a camera positioned to capture the detectable signal from the enzymatically activated detectable label; at least one focusing lens (e.g., a collimating lens) interposed between the light source, the detector, and the lateral-flow chromatographic assay cassette; and an interpretive algorithm stored in a computer readable format and electronically coupled to the testing device, wherein the interpretive algorithm is configured to convert the detectable signal from the enzymatically activated detectable label to a numerical value for quantification of at least one of the amount or the activity of at least one enzyme in the sample or the amount of an enzyme substrate in the sample; the lateral-flow chromatographic assay cassette further includes means for calibrating a response of the enzymatically activated detectable label to a reaction between the enzyme and the substrate, wherein the means for calibration includes at least one of: a lateral-flow chromatographic assay cassette that includes at least a first calibration standard and a second calibration standard configured to provide at least a two-point calibration curve; or a lateral-flow chromatographic assay cassette that includes a test strip and a separate calibration strip cassette, wherein the calibration strip includes an enzymatically activated detectable signal configured to provide a known response to a known amount of the enzyme; and the interpretive algorithm is further configured to (i) calculate a calibration curve and then (ii) convert the detectable signal from the enzymatically activated detectable label to a numerical value for quantification of the amount or the activity of at least one enzyme in the sample.

Also described herein are devices and methods for performing point of care diagnostic tests for detecting and quantifying at least one of the activity level or the concentration of an enzyme or a biochemical analyte in a biological sample. The devices and methods are configured to quantify at least one of the activity level or the concentration of at least one enzyme or an enzyme substrate in a biological sample (e.g., a body fluid) via an enzymatic reaction. For example, enzymatic degradation of a substrate can be used either to determine the activity or concentration of an enzyme in a sample or to determine the concentration of the substrate in a sample. Examples described herein are testing devices that can be used to provide rapid, accurate, affordable laboratory-quality quantitative testing at the point of care. Such devices are designed to eliminate or replace expensive, centralized clinical testing equipment and technical personnel. Such devices include automated data reporting and decision support.

In one example described herein, an enzyme-based assay system is disclosed. Such an enzyme based assay system can be used, for example, for quantification of an amount or an activity of an enzyme in a sample and/or for quantification of an amount a substrate in a sample. The system includes a lateral-flow chromatographic assay cassette configured for assaying a reaction involving an enzyme and a substrate, a testing device with data collection and data analysis capabilities that is configured to interface with the lateral-flow chromatographic assay cassette, and an interpretive algorithm stored in a computer readable format and electronically accessible by the testing device.

In another example described herein, the interpretive algorithm is configured to convert a detectable signal from an enzymatically activated detectable label to a numerical value for quantification of at least one of the amount or the activity of at least one enzyme in the sample or the amount of an enzyme substrate in the sample.

In another example described herein, the lateral-flow chromatographic assay cassette includes means for calibrating a response of the enzymatically activated detectable label to a reaction between the enzyme and the substrate, and the interpretive algorithm is further configured to (i) calculate a calibration curve and then (ii) convert the detectable signal from the enzymatically activated detectable label to a numerical value for quantification of the amount or the activity of at least one enzyme in the sample. In one example, the means includes a lateral-flow chromatographic assay cassette that includes at least a first calibration standard and a second calibration standard configured to provide at least a two-point calibration curve. In another example, the means includes a lateral-flow chromatographic assay cassette that includes a test strip and a separate calibration strip cassette, wherein the calibration strip includes an enzymatically activated detectable signal configured to provide a known response to a known amount of the enzyme.

In the case of the enzyme-based assay system for quantification of an amount or an activity of an enzyme in a sample, the lateral-flow chromatographic assay cassette includes a sample application zone in fluid communication with a test zone via a fluid transport matrix, wherein a substrate having an enzymatically-cleavable detectable label is immobilized in the test zone and the enzyme is in a mobile phase. The test zone lateral-flow chromatographic assay cassette further includes at least a first calibration standard and a second calibration standard configured to provide at least a two-point calibration curve for the enzyme-based assay system.

In the case of the enzyme-based assay system for quantification of an amount a substrate in a sample, the lateral-flow chromatographic assay cassette includes a sample application zone in fluid communication with a test zone via a fluid transport matrix. An enzyme specific to the substrate and an enzymatically activated detectable label that is configured to develop a detectable signal in response to enzymatic cleavage of the substrate are immobilized to the fluid transport matrix and the substrate is in the sample. The test zone further includes at least a first calibration standard and a second calibration standard configured to provide at least a two-point calibration curve for the enzyme-based assay system.

The lateral-flow chromatographic assay cassette includes a sample application zone in fluid communication with a test zone via a fluid transport matrix, wherein a substrate having an enzymatically-cleavable detectable label is immobilized in the test zone and the enzyme is in a mobile phase, and wherein the test zone further includes at least a first calibration standard and a second calibration standard configured to provide at least a two-point calibration curve for the enzyme-based assay system.

The testing device includes a testing apparatus that is configured for collecting data from the lateral-flow chromatographic assay cassette. In one example the testing apparatus is physically coupled to the testing device and the testing apparatus couples the lateral-flow chromatographic assay cassette to the testing device in proximity to a light source, the light source being capable of transmitting at least one wavelength of light configured to yield a detectable signal from the enzymatically activated detectable label, and a detector positioned to capture the detectable signal from the enzymatically activated detectable label. In another example, the testing apparatus is a stand-alone, albeit hand held, device that includes its own light source, optics, power source, data capture capabilities, and the like. In such an example, the testing apparatus may be configured to collect assay data from an assay cassette and transfer it to the testing device for analysis and reporting.

In yet another example, a method is disclosed. The method includes (1) providing a lateral-flow chromatographic assay cassette as described above, wherein the lateral-flow chromatographic assay cassette is configured for at least one of assaying the concentration or activity of an enzyme in the sample or for assaying the concentration of a substrate in a sample, and (2) providing a testing device as described above having data collection and data analysis capabilities.

In one example, the assay further includes (3) applying a liquid sample to the lateral-flow chromatographic assay cassette, wherein the liquid sample includes at least one enzyme, (4) inserting the lateral-flow chromatographic assay cassette into the testing apparatus, (5) illuminating the lateral-flow chromatographic assay cassette to yield a first detectable signal from the enzymatically activated detectable label, (6) allowing enzymatic cleavage of the detectable label from the substrate to proceed for a period of time, (7) illuminating the lateral-flow chromatographic assay cassette to yield a second detectable signal from the detectable label, wherein the second detectable signal is reduced relative to the first detectable signal in proportion to the concentration or activity of the enzyme in the liquid sample, and (8) querying an interpretive algorithm stored in a computer readable format accessible by the testing device.

In another example, the method further includes (3) applying a liquid sample to the lateral-flow chromatographic assay cassette, wherein the liquid sample includes at least one substrate, (4) inserting the lateral-flow chromatographic assay cassette into the testing apparatus, (5) illuminating the lateral-flow chromatographic assay cassette to yield a detectable signal from the enzymatically activated detectable label, and (6) querying an interpretive algorithm stored in a computer readable format accessible by the testing device.

In one example, a product of enzymatic cleavage of the substrate interacts with the enzymatically activated detectable label to yield the detectable signal. In another embodiment, a product of enzymatic cleavage of the substrate is linked development of the detectable signal from the enzymatically activated detectable label through at least one additional enzymatic reaction.

These and other objects and features of the present invention will become more fully apparent from the following description and appended claims, or may be learned by the practice of the invention as set forth hereinafter.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only illustrated embodiments of the invention and are therefore not to be considered limiting of its scope. The invention will be described and explained with additional specificity and detail through the use of the accompanying drawings in which:

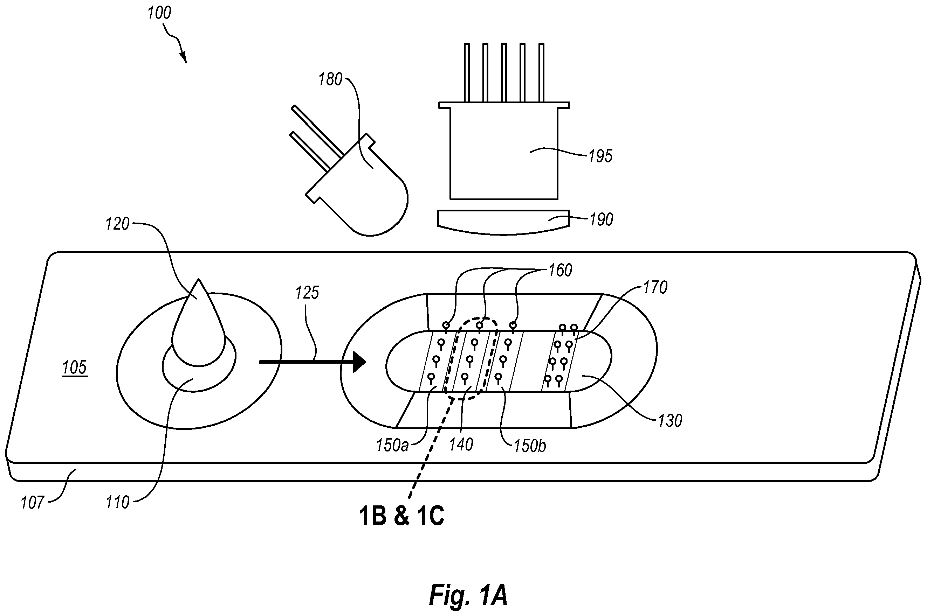

Figure 1A illustrates a perspective view of an enzyme-based diagnostic test system, according to one embodiment of the present disclosure;

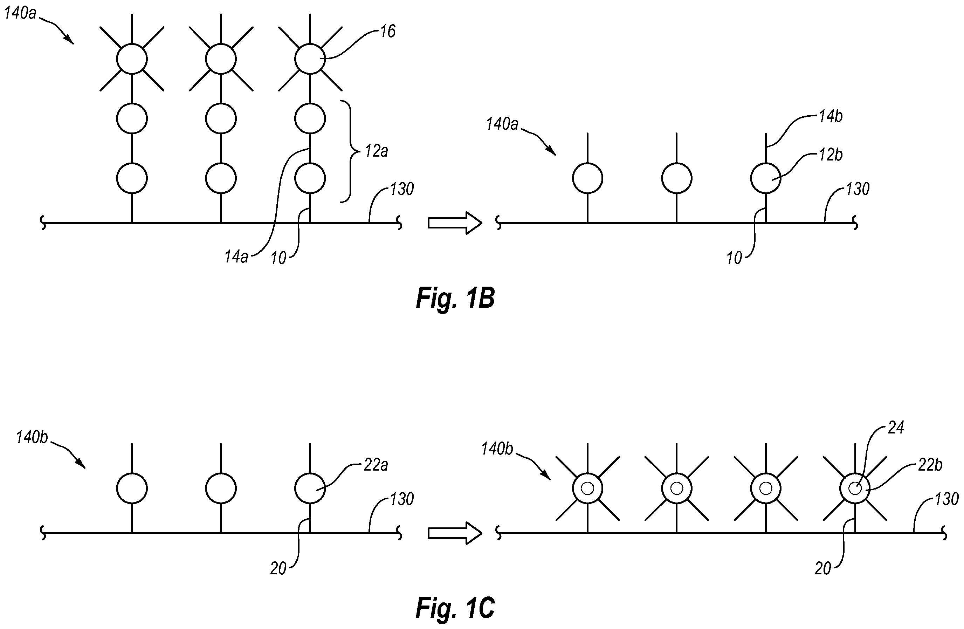

Figure 1B illustrates a detailed view of a portion of the enzyme-based diagnostic test system, according to one embodiment of the present disclosure; replace expensive, centralized clinical testing equipment and technical personnel. Such devices include automated data reporting and decision support.

In one embodiment, an enzyme-based diagnostic test system is disclosed. The system includes a lateral-flow chromatographic assay cassette and a testing device that includes data collection and data analysis capabilities. The testing device is configured to interface with and analyze output of the lateral-flow chromatographic assay cassette.

### I. Diagnostic Test Systems

Referring to Figure 1A, perspective view of an enzyme-based diagnostic test system 100 is illustrated. The enzyme-based diagnostic test system 100 includes a lateral-flow chromatographic assay cassette 105 and means for collecting assay data from the lateral-flow chromatographic assay cassette 105.

The lateral-flow chromatographic assay cassette 105 includes a plastic housing 107 containing a test strip, which is generally a plastic strip laminated with porous material that permits lateral flow of liquid. The illustrated lateral-flow chromatographic enzymatic assay cassette 105 includes a sample application zone 110 and an analysis zone 130.

When a sample 120 is applied to the lateral-flow chromatographic enzymatic assay cassette 105 at the sample application zone 110, the sample 120 diffuses through the strip in flow direction 125 toward the analysis zone 130. In the embodiment illustrated in Figure 1A, the analysis zone 130 includes a test line 140 and at least first and second calibration standard lines 150a and 150b.

The analyte(s) of interest (e.g., enzyme(s) or enzyme substrate(s)) and the first and second calibration standards can be detected on their various target lines, 140, 150a, and 150b, respectively, with various reporters. The reporters 160 for each of the various target lines, 140, 150a, and 150b, may be the same or different. Examples of suitable reporters include, but are not limited to, visible and fluorescent dyes, gold nanoparticles, silver nanoparticles, titanium nanoparticles, europium fluorophores, quantum dots, latex beads, enzymes, and the like. Quantum dots are nano-scale materials that can produce excited emission at particular wavelengths depending on their size and shape. Quantum dots can be used in enzymatic assays where dyes have traditionally been used. However, quantum dots are generally superior to traditional organic dyes on several counts: quantum dots are typically much brighter that organic dyes (owing to their high extinction coefficients combined with a comparable quantum yield to fluorescent dyes) as well as their stability (i.e., much less photobleaching). For example, it has been estimated that quantum dots are 20 times brighter and 100 times more stable than traditional fluorescent reporters.

Emission from the various reporters can be excited by a number of sources. In the illustrated embodiment, an LED light source 180 is used illuminate the analysis zone 130 of the lateral flow assay cassette 105. Illumination by the light source 180 may produce a detectable signal that includes at least one of emission (e.g., fluorescence), color, reflectance, diffuse scattering (i.e., scattering and absorbance), elastic light scattering, chemiluminescence, chemifluorescence, transmission, or absorbance from the reporters. A lens 190 (e.g., a collimating lens) and a detector (e.g., a CCD or CMOS camera) are used to collect data from the reporters and the first and second calibration standards.

When the sample 120 is applied to the diffusion strip of the lateral-flow chromatographic assay cassette 105, the liquid in the sample carries the analyte of interest through the diffusion strip in flow direction 125 into the analysis zone 130. Depending on the experiment being run, the analyte of interest may be an enzyme or an enzyme substrate. The first and second calibration standard lines 150a and 150b are selected to provide a detectable signal that correlate to non-zero concentration and/or activity values of the analyte of interest. For example, the first and second calibration standard lines 150a and 150b may include a material pre-bound to the diffusion strip of the lateral-flow chromatographic assay cassette 105. In response to illumination by the light source, the reporter 160 associate with each of lines 140, 150a, and 150b provides a signal that can be used to calculate a calibration curves and, in turn, determine the concentration and/or the activity level of the analyte of interest in the sample 120. A more detailed discussion of methods for deriving analyte concentration from the data of the first and second calibration standards 150a and 150b and the test line 140 is discussed in greater detail elsewhere herein.

Figures 1B and 1C illustrate methods that may be used to detect the activity or concentration of an enzyme in a sample (Figure 1B) or to enzymatically detect the concentration of an enzyme substrate in a sample (Figure 1C).

Referring to Figure 1B, a situation is illustrated where an enzyme being assayed is in a sample applied to assay cassette 105 and the enzyme's substrate is immobilized to the cassette 105. When the sample 120 is applied to the lateral-flow chromatographic assay cassette 105, the enzyme in the sample diffuses through the fluid transport matrix 130 of the lateral-flow chromatographic assay cassette 105. The enzyme is able to diffuse to the test line 140a where the enzyme can encounter the immobilized substrate 12a. For example, the substrate 12a may be immobilized to the fluid transport matrix 130 by a covalent linkage 10. In the illustrated embodiment, the substrate 12 is coupled to a detectable label 16; the detectable label 16 is cleavable from the substrate in response to enzymatic cleavage of cleavable bond 14a. Prior to degradation of the substrate, the detectable label 16 provides a first signal. In response to enzymatic cleavage of the substrate 14b, the detectable label is lost from the line of substrate. The concentration or activity of the enzyme is calculated as a function of the loss of signal from the substrate line 140 as a function of time.

Referring to Figure 1C, a situation is illustrated where an substrate being assayed is in a sample applied to assay cassette 105 and an enzyme that can break down the substrate is immobilized to the cassette 105. In this instance, the substrate is not detected directly. Instead, a product of breakdown of the substrate by one or more linked enzymatic reactions is able to interact with an enzymatically activated detectable label in such a way that renders the reporter detectable.

When the sample 120 is applied to the lateral-flow chromatographic assay cassette 105, the substrate, which is in the sample 120, diffuses through the fluid transport matrix 130 of the lateral-flow chromatographic assay cassette 105 where the substrate can encounter an immobilized enzyme (not shown) that can degrade the substrate, producing a breakdown product 24. In the absence of the breakdown product 24, the reporter 22a does not produce a detectable signal. In contrast, when the breakdown product 24 interacts with the reporter, the reporter is modified 22b, thus producing a detectable signal (e.g., phosphorescence, fluorescence, color change, etc.). The product of enzymatic breakdown of the substrate may interact directly with the reporter, or one or more additional enzymatic reactions may be linked to the first enzymatic reaction in order to produce a product that can interact with the reporter.

Suitable examples of enzymes that can be assayed using the devices and methods described herein include, but are not limited to, alanine aminotransferase, aspartate aminotransferase, amylase, lipase, gamma glutamyl transpeptidase, alkaline phosphatase, lactate dehydrogenase, acid phosphatase, aldolase, and glucose-6-phosphate dehydrogenase.

Suitable examples of enzymatic substrates that can be assayed using the devices and methods described herein include, but are not limited to, creatinine, uric acid, bilirubin, and phenylalanine, beta hydroxyl butyrate, alpha keto gluterate, lactic acid, ammonia, bicarbonate, bile acids, ethanol, glucose, cholesterol, triglycerides.

For example, an assay for creatinine may involve several linked enzymatic reactions. A method may include creatininase, creatinase, sarcosine oxidase, and a peroxidase. The hydrogen peroxide liberated in the sarcosine oxidase reaction is used by the peroxidase to produce a colored substance that can be measured spectrophotometrically or fluorimetrically. Uric acid, bilirubin, phenylalanine, and other metabolites may be detected using similar schemes.

Lateral-flow enzymatic assay cassettes may be adapted for assaying a number of different analyte types. For example, enzymatic assay cassettes have been adapted or may in the future be adapted for blood glucose testing, metabolic testing (e.g., thyroid stimulating hormone), blood gas and electrolytes analysis, rapid coagulation testing, rapid cardiac markers diagnostics, drugs of abuse screening, urine testing, pregnancy testing, fecal occult blood analysis, food pathogen screening, complete blood count ("CBC"), hemoglobin diagnostics, infectious disease testing (e.g., a multi-analyte rapid diagnostic test for detecting malaria infection), cholesterol screening, hormone testing, cardiac pulmonary, gastroenterology, urology, nephrology, dermatology, neurology, pediatrics, surgical, public health, and veterinary and plant pathology testing, combinations thereof, and the like.

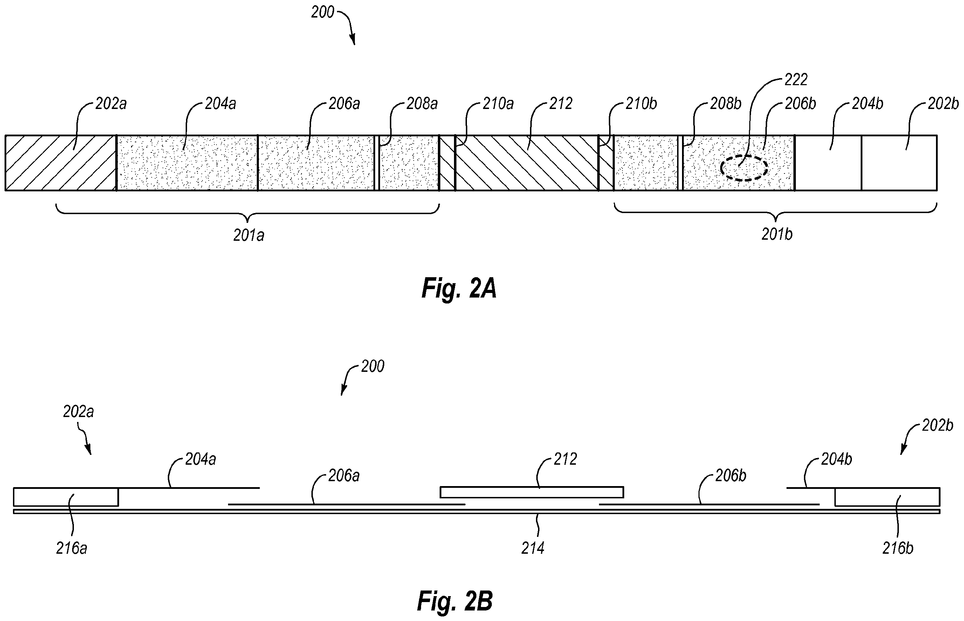

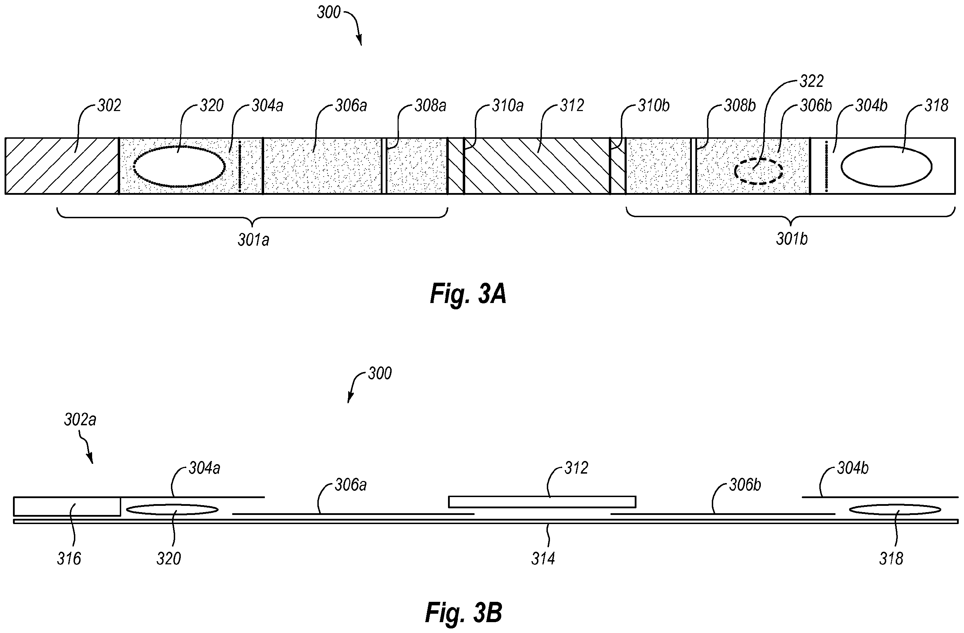

In addition to the foregoing, another embodiment of a lateral flow enzymatic assay cassette is described. Examples of such lateral flow enzymatic assay cassettes are shown at 200 in Figures 2A and 2B and at 300 in Figures 3A and 3B. In the lateral flow enzymatic assay cassettes 200 and 300, a test sample (i.e., a sample containing an unknown concentration of an analyte of interest (e.g., an enzyme or an enzyme substrate)) may be run in parallel with a calibration standard (i.e., a sample containing a known concentration of the analyte of interest). The response to the known concentration of the analyte of interest in the calibration standard on the lateral flow enzymatic assay device may be used to generate a calibration curve that can be used to quantify the amount of the analyte of interest in the test sample.

Such an arrangement may provide superior results. For example, the test and calibrations strips of such cassettes may be manufactured side-by-side under substantially equal temperature and humidity conditions. As a result, it is generally the case that the test and calibrations strips each have the same amount an enzymatically activated detectable label immobilized thereon and that the enzymatically activated detectable label on each will react substantially the same. Also, because the test and calibration assays are run in parallel, the test and calibration results are generally unaffected by factors like temperature and humidity. This is generally not the case if the test and calibration assays are run at separate times on strips that may have been manufactured at different times. Likewise, because the test and calibration assays are run in parallel, the cassettes and a reader device, if used, are calibrated for each assay run on each cassette, which is believed to provide more reliable quantitative results.

The lateral flow enzymatic assay cassette 200 illustrated in Figures 2A and 2B includes a base 214 that includes a test strip 201a and a calibration strip 201b. The test strip 201a includes a sample application zone 202a with a sample collection pad 216a, a conjugate pad 204a, a test assay strip 206a (e.g., a nitrocellulose ("NC") membrane), and an absorbent pad 212. Likewise, the calibration strip 201b includes a sample application zone 202b with a sample collection pad 216b, a conjugate pad 204b, a calibration strip 206b, and the absorbent pad 212. Each of the test assay strip 206a and the calibration strip 206b include at least one an enzymatically activated detectable label 208a and 208b that can specifically interact with the analyte of interest for detection. In one embodiment, the sample pad 212 may include flow indicator lines 210a and 210b (e.g., a water soluble dye) that indicate whether or not sample has successfully diffused through the test strip 201a and the calibration strip 201b.

In the illustrated embodiment, the test 201a and calibration strips 201b are run in opposite directions (i.e., both the test sample and calibration standard flow toward absorbent pad at the center of the cassette). In other embodiments, the test and calibration strips may be arranged such that the test sample and calibration standard flow parallel to one another. Such an embodiment may, for example, include a divider arranged between the test assay strip and the calibration assay strip.

The lateral flow enzymatic assay cassette 300 illustrated in Figures 3A and 3B is similar to the cassette 200 of Figures 2A and 2B. The lateral flow enzymatic assay cassette 300 includes a base 314 that includes a test strip 301a and a calibration strip 301b. The test strip 301a includes a sample application zone 302a with a sample collection pad 316, a conjugate pad 304a, a test assay strip 306a (e.g., a nitrocellulose ("NC") membrane), and an absorbent pad 312. In addition, the test strip 301a includes a sachet 320 (e.g., a blister pack) of buffer that can be used to chase (i.e., wash) a test sample through the conjugate pad 304a and the assay strip 306a toward the absorbent pad 312.

In contrast to the cassette 200 of Figures 2A and 2B, the cassette 300 omits a calibration standard application zone and instead includes a standard solution sachet 318 that contains a known volume of a solution that contains a known amount of at least one analyte of interest. When the a standard solution sachet 318 is pierced at the time of use, the solution wicks through the conjugate pad 304b and the calibration strip 306b toward the absorbent pad 312. Each of the test assay strip 306a and the calibration strip 306b include at least one enzymatically activated detectable label 308a and 308b that can specifically interact with the analyte of interest for detection. The characteristics of the standard solution sachet 318 can be used to test for quantitative delivery of the calibration standard onto the calibration strip 306b and to test the response of the enzymatically activated detectable label 308b to the analyte of interest. In one embodiment, the sample pad 312 may include flow indicator lines 310a and 310b (e.g., a water soluble dye) that indicate whether or not sample has successfully diffused through the test strip 301a and the calibration strip 301b.

In one embodiment, the sample pad 216a, 216b, or 316 may be configured to absorb and dispense a predetermined amount of a fluid from the fluid that is applied thereto. That is, the sample pad 216a, 216b, or 316 may be fabricated from an absorbent-type material that may saturated with fluid and then when, for example, the sample pad 216a, 216b, or 316 is compresses or squeezed, the sample pad 216a, 216b, or 316 can dispense a predetermined amount of a fluid therefrom. In one embodiment, the sample pad 216a, 216b, or 316 may be made of cellulose, glass fiber or other material where the fluid sample is applied to the lateral flow device and, if necessary modifies it to improve the results of the assay. This might be by modifying pH, filtering out solid components, separating whole blood constituents, adsorbing out unwanted antibodies or some other test specific variable.

For some applications, the sample pad 216a, 216b, or 316 may be pretreated by dipping it into a specific buffer containing a mix of a solution comprised of soluble proteins, surfactants/detergents, and other polymers. These may allow for a steady flow and prevent nonspecific binding of sample components to the pad 216a, 216b, or 316.

In some embodiments, the sample may be added to the sample pad 216a, 216b, or 316 by collecting a liquid sample (e.g., blood, urine, or saliva) and adding a selected volume of the sample to the sample pad. In other embodiment, the sample may be added to the sample pad 216a, 216b, or 316 by soaking the pad with a fluid sample. For example, the sample pad 216a, 216b, or 316 may be soaked with saliva by inserting the sample collection pad 216a, 216b, or 316 end of the device 200 or 300 into the mouth to collect a saliva sample.

In one embodiment, the conjugate pad 204a, 204b, 304a, 304b is made of a non-absorbent material such as fiberglass pad, polyester, rayon or a similar material. The conjugate pad 204a, 204b, 304a, 304b is typically fabricated from a synthetic material (at least when using a gold conjugate) to ensure the efficient release of its contents.

As its name implies, the assay's detection conjugate (e.g., colloidal gold) is dried down and held in place in the conjugate pad 204a, 204b, 304a, 304b until a liquid test sample is applied to the sample pad. The liquid from the sample, by capillary action moves into the conjugate pad 204a, 204b, 304a, 304b, re-hydrates the dry conjugate and allows the mixing of the sample with the conjugate. The complex of conjugate and analyte then moves into and up the assay strip 206a, 206b, 306a, 306b. Pretreatment of the conjugate pad 204a, 204b, 304a, 304b helps to ensure the conjugate releases at the proper rate and enhances its stability. The pretreatment is performed in the same way as with the sample pad 216a, 216b, or 316.

In one embodiment, the at least one capture binding moiety 208a, 208b, 308a, 308b may be added to the test or calibration strips with a dispenser that gently slides a soft capillary tube across the membrane. A dispenser pump releases a constant volume of the reagents down the length of the membrane. This system is simple, easy to use, and low cost. They can be somewhat cumbersome in large scale manufacturing and many systems require a technician to constantly feed the nitrocellulose cards and to monitor reagent levels as well as the quality of the test and control lines.

An alternative method of applying the at least one capture binding moiety 208a, 208b, 308a, 308b includes a non-contact aerosol system. These sprayers dispense solutions in controlled ultrafine, ultra- small volume aerosols. These devices project very fine droplets of reagent onto the membrane and overlap the drops to create a continuous line. Spraying offers much more control of the reagent application, but it also adds capital expense and increases the complexity of strip manufacturing. These devices are more appropriate in very large scale manufacturing or when a reader with tight tolerances will be used to analyze the lateral flow test strips.

In the foregoing, addition of one line of the at least one capture binding moiety 208a, 208b, 308a, 308b onto each of the test or calibration strips is discussed. However, one will appreciate that a cassette 200 or 300 may include multiple test and control lines that may each be configured to interact with a different analyte of interest.

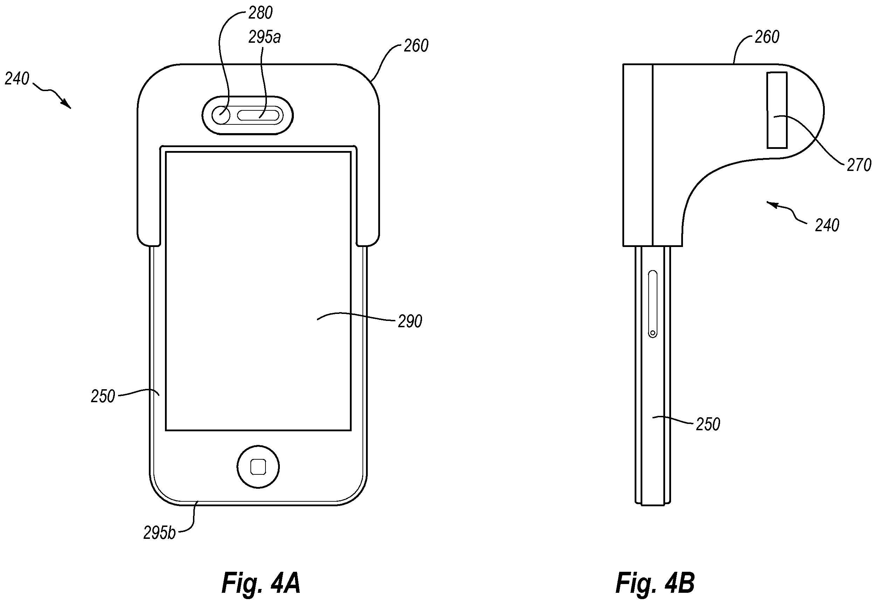

Referring now to Figures 4A and 4B, plan and side views of an enzyme-based diagnostic test system 240 are illustrated. The illustrated enzyme-based diagnostic test system 240 includes a testing device 250 and a testing apparatus 260.

In the illustrated embodiment, the testing device 250 is an iPhone. However, the testing 250 device can be essentially any cell phone device, digital camera device, or a similar device that has an onboard camera/image capture function, data collection and analysis capabilities, data and results display capabilities, and, preferably, the ability to communicate with one or more remote computer networks through a cellular telephone network for data upload, querying a data analysis algorithm, querying a decision support algorithm, and the like. In the illustrated embodiment, the testing device 250 includes a front-directed camera 280, a back-directed camera (not shown) that is directed into the testing apparatus, a display screen 290, and audio input and output ports 295a and 295b. The display screen 290 can be used for display of data and results. In addition, the display screen 290 may include touchscreen capabilities that can be used for input of data or commands.

In one embodiment, the testing apparatus 260 is designed to be securely coupled to the testing device 250. For example, the testing apparatus 260 may be designed to fit a specific class or brand of testing devices. The testing apparatus includes a cassette port 270 that is designed to allow an assay device, such as a lateral flow enzymatic assay cassette 105 (see Figure 1A), to be inserted into the testing apparatus 260. Additionally, an interior portion of the testing apparatus 260 may be painted with a flat black color so as to avoid extraneous and reflected light. In addition, the testing apparatus 260 includes a number of internal components (e.g., i/o ports, power ports, light source(s), lens(es), light conducting media, etc.) that are designed to transform the testing device 250 into a device that can be used to collect and analyze data produced by an assay device, such as the lateral flow enzymatic assay cassette 105 (see Figure 1A).

While the testing apparatus 260, is shown fitted to the testing device 250, one will appreciate that they testing apparatus can be configured as a separate unit that includes its own light source, power supply, optics, data capture capabilities, and the like. In such an embodiment, the testing apparatus may be configured to collect assay data from an assay cassette and transfer it (e.g., by a wired or wireless connection, by Bluetooth™, or the like) to the testing device for analysis and reporting.

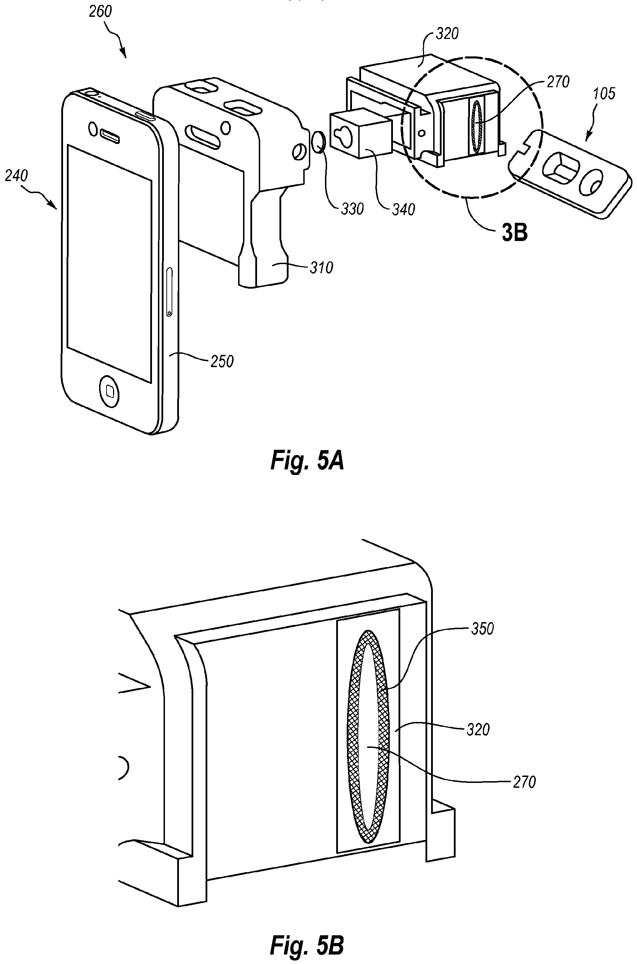

Referring now to Figure 5A, Figure 5A illustrates an exploded view of the enzyme-based diagnostic testing system 240 that is illustrated in Figures 4A and 4B. As can be seen in the exploded view, the testing apparatus 260 includes a main body housing 310 and an assay housing 320.

The main body housing 310 is primarily designed to mate cleanly with the testing device 250; preferably forming a light-tight seal with the testing device 250. For example, the main body housing 310 may be shaped such that the testing device 250 can be slid into the main body housing 310 such that the testing device 250 clicks into or otherwise securely mates with the main body housing 310. The main body housing 310 may also include one or more gaskets, seals, and the like that allow the testing device to form a secure and light-tight seal with the main body housing 310. Additional features of the main body housing 310 will be discussed below.

The assay housing 320 is fixedly coupled to the main body housing 310. In the illustrated embodiment, the assay housing 320 includes a cassette port 270 that is configured such that a lateral flow enzymatic assay cassette 105 can be inserted into the assay housing 320. In addition, the assay housing 320 in the in the illustrated embodiment includes a lens that is interposed between the testing device's 250 back-directed camera (not shown) and the lateral flow enzymatic assay cassette 105. Likewise, an optical fiber device or light pipe 340 that is capable of transmitting light either to the lateral flow enzymatic assay cassette 105 from the hand held device's 250 light source (not shown), from the lateral flow enzymatic assay cassette 105 to the hand held device's 250 back-directed camera (not shown), or both.

While the hand held device's 250 light source (not shown) can be used to illuminate the lateral flow enzymatic assay cassette 105, the enzyme-based diagnostic testing system 240 may also include one or more additional light sources that can be housed in either the assay housing 320 or the main body housing 310. Suitable examples of light sources can include, but are not limited to a camera flash, an autofocus illuminator on a camera, an LED light, an incandescent lamp, or a gas-discharge lamp. For example, the light source can come from micro-LED lamps that are included in the assay housing 320. The micro-LEDs can be selected to emit certain wavelengths that are adapted for one or more assay conditions. The micro-LEDs can be powered by drawing electrical power from the battery of the testing device 250. In addition, either the assay housing 320 or the main body housing 310 may be configured such that ambient light or sunlight can be used to illuminate the lateral flow enzymatic assay cassette 105.

In one embodiment, at least one wavelength filter may be interposed between the light source and the lateral-flow chromatographic enzymatic assay cassette 105. For example, if the assay is a fluorescent assay, then the wavelength filter may be used to yield a specific wavelength of light from the light source to excite fluorescent emission from the assay system. Likewise, certain colored dyes may yield a better signal when excited by selected wavelengths of light.

In one embodiment, the lens 330 (e.g., a collimating lens) may be used for focusing the light source on the lateral-flow chromatographic enzymatic assay cassette 105. For example, the lens 330 may be used to increase the amount of incident light impinging on the lateral-flow chromatographic enzymatic assay cassette 105. For instance, the purpose of the lens 330 may be to bring the focal point of the camera of the testing device 250 (which is limited to about 6 inches or more) to less than 2 centimeters. This allows for a smaller overall package and produces a finer image that prevents the use of convoluting a blurry picture using Fourier transforms in order to produce a usable data that can be analyzed. Furthermore, with a multi-analyte detection assay (e.g., two calibration standard lines and a test sample line), the finer image will prevent overlap of the target lines to improve sensitivity and accuracy. In another example, a focusing apparatus may be used to focus ambient light or sunlight on the analysis zone of the lateral-flow chromatographic enzymatic assay cassette 105.

In some embodiments, the assay cover 320 may include a device that can allow the angle of the lateral-flow chromatographic enzymatic assay cassette 105 to be adjusted relative to the testing device 250 and a light source (not shown). By selectively modifying these angles, the lower detection limit of the assay can be extended, the signal to noise ratio can be improved, etc. In one embodiment, the device can be adjusted manually in order to choose an angle that optimizes detection limit, signal to noise, and the like. In another embodiment, the device can be coupled to a mechanical means, such as a servo motor or a gel-damped spring device that can allow the device to automatically sample a number of angles while the testing device 250 collects data from the lateral-flow chromatographic enzymatic assay cassette 105.

Referring now to Figure 5B, the assay housing 320 and the cassette port 270 are illustrated in greater detail. In the embodiment illustrated in Figure 3B, the cassette port 270 of the assay housing 320 includes a sealing gasket 350 disposed around the cassette port 270 that can seal the cassette port 270 when an assay cassette 105 is inserted therein so that ambient light does not leak into the housing 260. For example, if ambient light leaks into the housing 260, it could skew results. In addition, the cassette port 270 may include a springloaded flap (not shown) or similar means that can seal ambient light out of the housing 260 even when no cassette 105 is inserted into the cassette port 270.

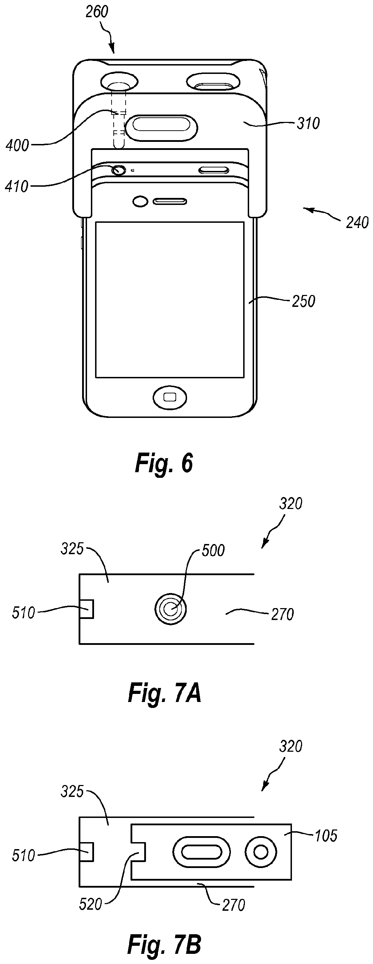

Referring now to Figures 6, 7A, and 7B, additional features of the testing apparatus 260 are illustrated.

Referring to Figure 6, an example of an indexing feature that can reliably align the testing apparatus 260 relative to the testing device 250 is illustrated. In the illustrated embodiment, the indexing feature includes a headphone jack 410 that is integrated into the housing body 310. When the testing device 250 is inserted into the housing body 310, the headphone jack 400 is positioned such that it can be inserted into the headphone port 410 of the testing device 250. It will be understood by persons having ordinary skill in the art that headphone jack 400 is but one example of an indexing feature and that additional indexing features can be employed without departing from the spirit of this discussion.

In addition to aligning the housing body 310 relative to the testing device 250, the headphone jack 400 can be used to draw electrical power from the testing device 250 in order to power components (e.g., one or more illumination devices) that are positioned in the housing 260. Likewise, the headphone jack 400 can be used for data transfer between the testing device 250 and components in the housing 260.

Referring now to Figure 7A, a target device 500 is illustrated. The target device 500 can be used to normalize/calibrate the response of at least one of the camera or the light source of the testing device. In one embodiment, the target device may located on an interior surface 325 of the assay housing 320 in close proximity to the cassette port 270 in an area that can be illuminated by a light source that will be employed for illumination of an assay cassette and viewable by a camera of a testing device that is going to be used to capture data from the cassette. For example, the target device may have a known color and/or color intensity that can give a known response for calibrating the light source and the camera. In addition, the target device 500 can be used to ensure that the light source and the camera are directed at the proper point when the testing device in inserted into the housing.

Referring now to Figures 7A and 7B, the assay housing 320 may further include a mechanical interlocking feature 510 that is positioned and configured to mate with a mechanical interlocking feature 520 on the assay cassette. For example, the mechanical interlocking features 510 and 520 may include tab and cut-out features that are designed to fit together. Such mechanical interlocking features 510 and 520 may be used to ensure that the cassette 105 is inserted in to the assay housing 320 in the proper orientation. In addition, such mechanical interlocking features 510 and 520 may be coupled to a disabling feature that can shut down the device if an incompatible cassette is inserted into the housing 320 or if the cassette is inserted in the wrong orientation. This can, for example, be an important safety feature because it prevents the device from reading the wrong portion of the cassette and giving an erroneous reading as a result.

### II. Methods for Detecting At Least One Analyte of Interest in a Sample

In one embodiment, a method for quantification of a concentration or an activity of an enzyme in a sample. The method includes (1) providing a lateral-flow chromatographic assay cassette as described above, wherein the lateral-flow chromatographic assay cassette is configured for assaying the concentration or activity of an enzyme in the sample, and (2) providing a testing device as described above having data collection and data analysis capabilities.

The assay further includes (3) applying a liquid sample to the lateral-flow chromatographic assay cassette, wherein the liquid sample includes at least one enzyme, (4) inserting the lateral-flow chromatographic assay cassette into the testing apparatus, (5) illuminating the lateral-flow chromatographic assay cassette to yield a first detectable signal from the detectable label, the first calibration standard, and the second calibration standard, (6) allowing enzymatic cleavage of the detectable label from the substrate to proceed for a period of time, (7) illuminating the lateral-flow chromatographic assay cassette to yield a second detectable signal from the detectable label, wherein the second detectable signal is reduced relative to the first detectable signal in proportion to the concentration or activity of the enzyme in the liquid sample, and (8) querying an interpretive algorithm stored in a computer readable format accessible by the testing device.

In another embodiment, a method for quantification of a concentration of a substrate in a sample is disclosed. The method includes (1) providing a lateral-flow chromatographic assay cassette as described above, assay cassette as described above, wherein the lateral-flow chromatographic assay cassette is configured for assaying the concentration of a substrate in the sample using an enzymatic reaction and an enzymatically activated detectable label that interacts with a product of enzymatic cleavage, and (2) providing a testing device having data collection and data analysis capabilities as described above.

The method further includes (3) applying a liquid sample to the lateral-flow chromatographic assay cassette, wherein the liquid sample includes at least one substrate, (4) inserting the lateral-flow chromatographic assay cassette into the testing apparatus, (5) illuminating the lateral-flow chromatographic assay cassette to yield a detectable signal from the reporter, the first calibration standard, and the second calibration standard, and (6) querying an interpretive algorithm stored in a computer readable format accessible by the testing device.

In one embodiment, a product of enzymatic cleavage of the substrate interacts with the reporter to yield the detectable signal. In another embodiment, a product of enzymatic cleavage of the substrate is linked development of the detectable signal from the reporter through at least one additional enzymatic reaction.

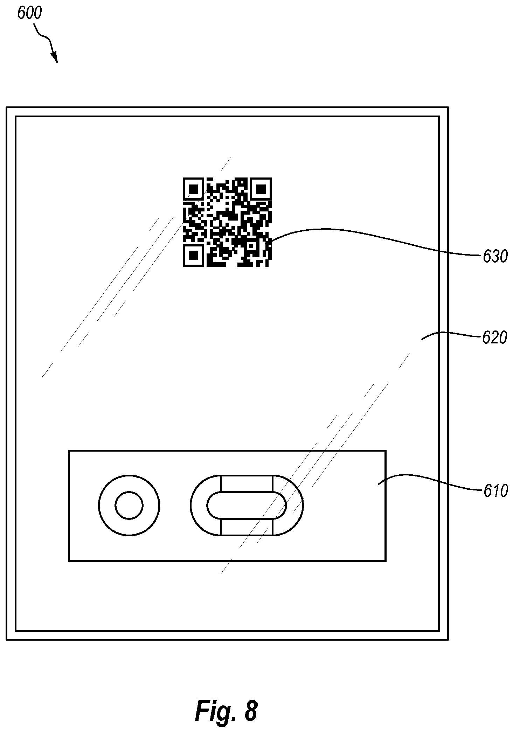

Referring now to Figure 8, an embodiment a packaging system 600 for providing the lateral-flow chromatographic assay cassette 610 is illustrated. The packaging system 600 includes a sealed package (e.g., a plastic-, foil-, or paper-based package) that can be used for containing, storing, or transporting the lateral-flow chromatographic assay cassette 610 in a clean and preferably sterile environment.

In addition, the packaging system 600 includes a tracking code 630. In the illustrated embodiment, the tracking code 630 is a QR code, which is a two-dimensional bar code. Two-dimensional bar codes, like QR codes, can be used to store far more information that can be stored in a conventional bar code. For example, a QR code can be used to store up -4300 alphnumeric characters (i.e., 0-9, A-Z, space, $, %, *, +, -, ., /, :, etc.). In one embodiment, the tracking code 630 can be read by the diagnostic testing system prior to initiating a test. The tracking code may be used to store information that is relevant to the test in a format that can be read by the device. For example, the tracking code 630 can be used for recording and then transmitting to the test system the values for the calibration standards used on the lateral-flow chromatographic assay cassette 610, manufacturer, date of manufacture, lot number for the lateral-flow chromatographic assay cassette 610, manufacturer, date of manufacture, and sample/results tracking.

In one embodiment, a single enzymatic assay device may contain multiple types of different enzymes or substrates that can be linked to different reporters (e.g., different colored quantum dots) so that multiple analytes can be assayed simultaneously. A single light source (e.g., an ultraviolet light) illuminates all the reporters (e.g., quantum dots) simultaneously, and the detector device (e.g., a digital camera) captures the emitted signals from multiple bands simultaneously.

In one embodiment, analytes of interest assayed on the lateral flow enzymatic assay cassettes described herein may be detected and quantified by elastic light scattering. The amount of light scattered from a selected region of a lateral flow enzymatic assay cassette (e.g., a capture band) is highly sensitive to the amount of material in a region illuminated by an incident light. In general, elastic light scattering, coupled with angle optimization, may be as much as 100 times more sensitive than comparable reflectance or fluorescence analysis. Other excitation/detection methods may include surface plasmon detection; Rayleigh scattering, reflectance, diffuse scattering, electrochemical detection, conductivity, fluorescence, magnetic, enzymatic, transmission, absorbtion, any other method which is based upon Beer's law, kinetic analysis (e.g., change in signal strength over time), and the like.

In one embodiment, a light source may be positioned at a certain angle to the lateral flow assay cassette and the detector (e.g., a detection fiber or a cell phone camera) or fiber (eventually the cellphone camera CCD). In one embodiment, the reporter(s) may be queried by taking a reading from each reporter and calculating the intensity of the scattered light. Signal intensity (i.e., the amount of scattered light that is detected) decreases as the concentration of the analyte of interest increases.

In an embodiment that includes a cell phone camera or the like, the camera's CCD will take an image. In one embodiment, the image will be taken with a red distance filter. In the image, the calibration standard lines and the test lines will be present. The digital image will then undergo digital image processing with a selected digital processing algorithm to produce a representative image of the color bands for the calibration standard lines and test simultaneously. For example, the digital processing algorithm may (1) identify the areas of interest (e.g., the test line and the at least two calibration standard lines) in the image taken of the lateral flow enzymatic assay cassette, (2) calculate an RGB value for each pixel in the image, (3) convert RGB format to xyz format, (4) convert xyz format to Lab color format, (5) assign a numerical value to each of the areas of interest (e.g., the test line and the at least two calibration standard lines), (6) calculate a calibration curve based on the numerical values obtained from the first and second calibration standard lines values, and (7) convert the numerical value for the test line into a concentration value for the analyte of interest in the sample.

In addition, internal controls, such as but not limited to, a control line (e.g., a fluorescent marker) to potentially eliminate or reduce variations in the final signal from manufacturing tolerances of the lateral flow assay cassette may be used to increase the robustness and reliability of the analysis. Additionally, analysis of the white portion of the lateral flow assay cassette may be used as an additional negative control to further improve reproducibility.

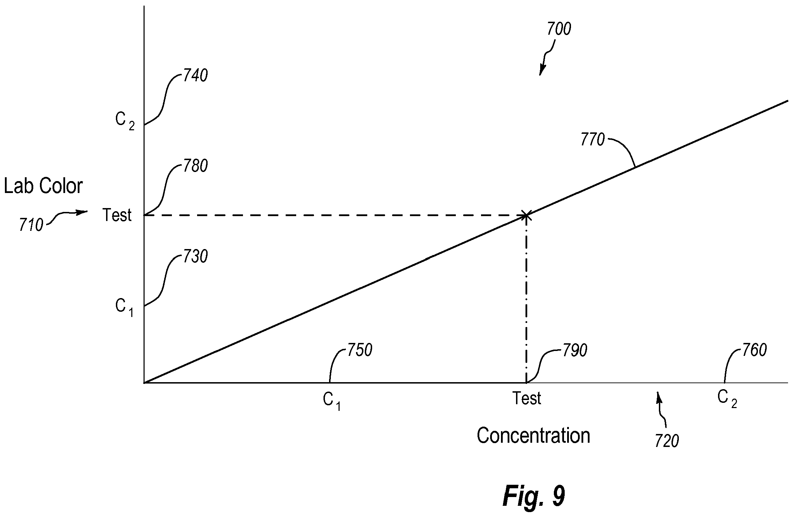

The digital processing algorithm is able to convert the numerical value for the test line into a concentration value because the at least two calibration standard lines are selected to provide numerical values that are proportional to non-zero concentration amounts for the analyte of interest. This relationship is clarified by reference to Figure 9, which shows a graph 700 with Lab value on the Y-axis and concentration on the X-axis. The first and second calibration standards have a known response that relates to known and, preferably, non-zero concentration values for the analyte of interest. Lab values for each of the first and second calibration standards 730 and 740 can be related to a concentration for each 750 and 760 by a simple relationship. By relating observed Lab color values to concentration values 750 and 760, a calibration curve 770 can be generated that can be used to calculate the concentration 790 of the analyte of interest in the sample based on the observed Lab color 780. One will of course appreciate that the calibration curve 770 can also be described by a mathematical formula and that the analysis algorithm may not actually generate a calibration curve, per se.

In one embodiment, the method may further include mixing the liquid sample with a dye conjugate prior to applying the sample to the lateral-flow chromatographic enzymatic assay cassette. In one embodiment, the dye conjugate is configured to interact with at least one of the analyte of interest or the ligand to provide a visual readout related to the presence or concentration of the analyte of interest in the sample. In one embodiment, the sample includes at least one control substance and at least one analyte of interest.

In one embodiment, the observation of the interaction of the at least one analyte of interest with the at least one ligand immobilized on the lateral-flow chromatographic enzymatic assay cassette may be timed by observing the appearance of at least one control substance. For example, a thyroid stimulating hormone ("TSH") assay may be read ∼10 minutes after a diluent is applied. By monitoring the position of the wave front or the appearance of the control line, it may be possible to eliminate the need to manually time the test. Likewise, by observing the timing of the appearance of a control, the most favorable time for reading the assay can be identified. These could include monitoring the movement of the mobile phase, monitoring the movement of the control substance, timing the movement of the mobile phase, taking sequential images of the test result, detecting when buffer is added, detecting when liquid has traveled the length of the membrane, and combinations thereof.

In one embodiment, the interpretive algorithm queried in the above described method may include one or more computer storage media having stored thereon computer executable instructions that, when executed by one or more processors of the detector device, implement a method for interpreting the numerical value related to the presence or amount or activity level of the at least one analyte present in the sample. In one embodiment, the computer implemented method may include (1) receiving a user initiated request to convert the visual signal readout of the enzymatic assay apparatus to a numerical value, (2) in response to the request, an act of identifying at least one visual signal readout of the enzymatic assay apparatus, (3) capturing at least one digital signal from the at least one visual signal readout of the enzymatic assay apparatus, (4) converting the at least digital signal to at least one numerical value, and (5) using the at least one numerical value to determine an amount or concentration or activity of at least one analyte present in the sample. This numerical value can then be displayed on a screen located on the detector device and/or stored, interpreted, or sent to a database.

In one embodiment, the computer implemented method may further include at least one of: (1) communicating with an electronic medical records system via a wireless communication channel, (2) uploading the amount or concentration of the at least one analyte present in the sample to the electronic medical records system, (3) querying a decision support algorithm, wherein the decision support algorithm uses the at least one numerical value to support a diagnosis of at least one condition in a subject and to suggest a course of treatment, or (4) adding the information to a public health database.

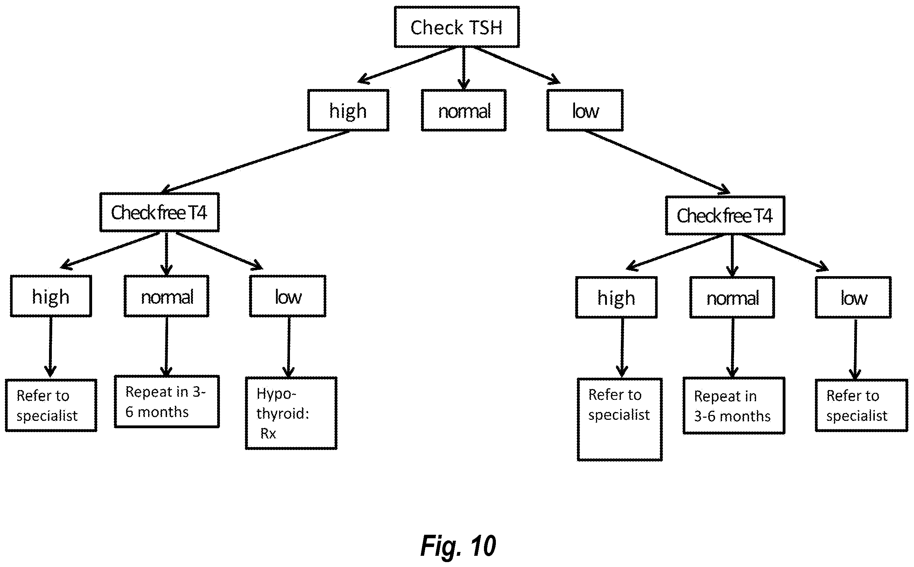

Figure 10 schematically illustrates the decisions that may be made or actions that may be taken in an example decision support algorithm for a thyroid stimulating hormone (TSH) test. At the first branch point, if TSH is normal then no action is taken. If TSH is low, a clinician will be directed to check free thyroxine (T4). If free T4 is normal, the algorithm directs that the test should be repeated in 3-6 months; if free T4 is high or low, the algorithm directs that the patient should be referred to a specialist. If at the first branch point TSH is high, the clinician will be directed to check free T4. If free T4 is normal, the algorithm directs that the test should be repeated in 3-6 months; if free T4 is high, the patient should be referred to a specialist; and if free T4 is low, the algorithm directs that the patient should receive a hypothyroid prescription.

In addition to the example described in reference to Figure 10, serum calcium is another example of a metabolite whose concentration can be determined enzymatically. For example, serum calcium levels can be measured using urea amidolyase (EC 3.5.1.45) from yeast species. The method is based on inhibition of the enzyme by calcium. The assay is described in detail in an article by Kimura et al. entitled "New enzymatic assay for calcium in serum," Clinical Chemistry, 42:8, pp. 1202-1205 (1996), the entirety of which is incorporated by reference.

A decision tree similar to the decision tree of Figure 10 is illustrated below in Table 1.

**Table 1**

| **Serum Calcium** | | | | | | |
|---|---|---|---|---|---|---|
| High | | | Normal | Low | | |
| Check parathyroid hormone (PTH) | | | | Check serum phosphate | | |
| High | Normal | Low | | High | Normal | Low |
| Evaluate for hyperparathyroidism | Consider malignancy, granulomatous disease, endocrine disease, familial, drugs | Excess vitamin D | | Check renal function and PTH | Check albumin, ionized calcium, and PTH | Check PTH and magnesium |

Embodiments of the present disclosure may comprise or utilize special purpose or general-purpose computing devices that include computer hardware, such as, for example, one or more processors and system memory, as discussed in greater detail below. Embodiments within the scope of the present invention also include physical and other computer-readable and recordable type media for carrying or storing computer-executable instructions and/or data structures. Such computer-readable recordable media can be any available media that can be accessed by a general purpose or special purpose computer system. Computer-readable media that store computer-executable instructions according to the invention are recordable-type storage media or other physical computer storage media (devices) that are distinguished from mere transitory carrier waves.

Computer-readable media that carry computer-executable instructions are transmission media. Thus, by way of example, and not limitation, embodiments of the invention can comprise at least two distinctly different kinds of computer-readable recordable media: computer storage media (devices) and transmission media.

Computer storage media (devices) includes RAM, ROM, EEPROM, CD-ROM or other optical disk storage, magnetic disk storage or other magnetic storage devices, or any other medium which can be used to store desired program code means in the form of computer-executable instructions or data structures and which can be accessed by a general purpose or special purpose computer and which are recorded on one or more recordable type medium (device).

A "network" is defined as one or more data links or communication channels that enable the transport of electronic data between computer systems and/or modules and/or other electronic devices. When information is transferred or provided over a network or another communications connection or channel (either hardwired, wireless, or a combination of hardwired or wireless) to a computer, the computer properly views the connection as a transmission medium. Transmissions media can include a network and/or data links which can be used to carry or desired program code means in the form of computer-executable instructions or data structures and which can be accessed by a general purpose or special purpose computer. Combinations of the above should also be included within the scope of computer-readable media.

Further, upon reaching various computer system components, program code means in the form of computer-executable instructions or data structures can be transferred automatically from transmission media to computer storage media (devices) (or vice versa). For example, computer-executable instructions or data structures received over a network or data link can be buffered in RAM within a network interface module (e.g., a "NIC"), and then eventually transferred to computer system RAM and/or to less volatile computer storage media (devices) at a computer system. Thus, it should be understood that computer storage media (devices) can be included in computer system components that also (or even primarily) utilize transmission media.

Computer-executable instructions comprise, for example, instructions and data which, when executed at a processor, cause a general purpose computer, special purpose computer, or special purpose processing device to perform a certain function or group of functions. The computer executable instructions may be, for example, binaries, intermediate format instructions such as assembly language, or even source code. Although the subject matter has been described in language specific to structural features and/or methodological acts, it is to be understood that the subject matter defined in the appended claims is not necessarily limited to the described features or acts described herein. Rather, the described features and acts are disclosed as example forms of implementing the claims.

Those skilled in the art will appreciate that the invention may be practiced in network computing environments with many types of computer system configurations, including, personal computers, desktop computers, laptop/notebook computers, message processors, hand-held devices, multi-processor systems, microprocessor-based or programmable consumer electronics, network PCs, minicomputers, mainframe computers, tablets, mobile telephones, PDAs, pagers, routers, switches, and the like. The invention may also be practiced in distributed system environments where local and remote computer systems, which are linked (either by hardwired data links, wireless data links, or by a combination of hardwired and wireless data links) through a network, both perform tasks. In a distributed system environment, program modules may be located in both local and remote memory storage devices.

In particular, one or more embodiments of the invention may be practiced with mobile consumer computing devices. Mobile consumer computing devices or more simply, mobile consumer devices, can be any of a broad range of computing devices designed or optimized for portability and for personal use. Mobile consumer devices can take a variety of forms, ranging from more traditional notebook and netbook computers to an emerging and rapidly growing market of handheld devices, including smart phones (e.g., the APPLE IPHONE, ANDROID phones, WINDOWS phones, SYMBIAN phones), tablet computers (e.g., the APPLE IPAD, ANDROID tablets), gaming devices (e.g., NINTENDO or PLAYSTATION portable gaming devices, the APPLE IPOD), multimedia devices (e.g., the APPLE IPOD), and combinations thereof. Many of these devices can enable rich user-interactivity by including combinations of output, input, and other sensory devices, such as touch- or pressure-sensitive displays (using capacitive or resistive technologies, for example), still and video cameras, Global Positioning System (GPS) receivers, magnetic compasses, gyroscopes, accelerometers, light sensors, proximity sensors, microphones, speakers, etc. These devices can also comprise a variety of communications devices, such as combinations of cellular modems (e.g., Global System for Mobile Communications (GSM), Code division multiple access (CDMA)), Wireless Fidelity (Wi-Fi) radios, Bluetooth radios, Near Field Communication (NFC) devices, etc. Many mobile consumer devices are expandable, such that a user can add new hardware and functionality not present during manufacture of the device. It will be appreciated that as the market for mobile consumer devices expands and develops, the functionality of these devices will also expand to utilize new and improved user-interaction devices and communications devices. The embodiments described herein are expansive and can also utilize any future developments in the field of mobile consumer devices.

### Example

The following Example describes an example of a test device that includes an iPhone and a test device coupled to the iPhone. The test device includes a slot for inserting a lateral flow assay cassette into the test device for reading and analysis by the iPhone.

There are a number of challenges associated with imaging the measurement cassette. The first is to fill the iPhone's camera frame with as much of the detection strip as possible. This suggests a short distance between the camera and cassette. The second challenge is to evenly illuminate the detection strip to make image processing easier. This requirement suggests a longer distance.

Generally, even illumination is the more challenging requirement. In one embodiment, a light pipe or a similar device may be interposed between the illumination source (e.g., the iPhone's flash or another light source that is included in the test device). Light pipes are commercially available in various configurations, such as, but not limited to, cylinders and rectangles. The rectangle shape has been tested and been found to work better than the cylindrical configuration. The physical dimensions of the rectangular light pipe are in the following document online: http://www.lumex.com/specs/LPB-R0112051S.pdf, the entirety of which is incorporated herein by reference.

As described above with respect to the Figures, the test device may include an accessory lens that is disposed between the camera's lens and the lateral flow assay cassette. The lens currently being tested has a 20mm focal length and 6mm diameter. This lens was ordered from Thorlabs.com with physical dimensions selectable in several formats from: http://www.thorlabs.us/thorProduct.cfm?partNumber=LA1700-A, the PDF version is: http://www.thorlabs.us/Thorcat/4400/4414-E0W.pdf, the entireties of which are incorporated herein by reference. A 30mm focal length should be a good value for filling the iPhone camera's frame and achieving even illumination of the detection strip. A focal length of 60mm is also an interesting choice since the iPhone may not need a second lens. However, this may potentially limit sensitivity in the final measurement.

One will of course appreciate that either the light pipe or the lens may include one or more light filters that allow selective illumination of the detection strip and/or detection of selection wavelengths of light from the detections strip. Likewise, the test device may include one or more light sources that emit selected wavelengths of for illumination of the detection strip. Analysis of images or a detection strip configured for detection of TSH with colloidal gold with a properly configured light pipe show dips in reflectivity in all three color channels (red, blue, green). With a proper exposure, there is a greatest difference in the green channel, corresponding to the 580nm peak in the reflectance spectrum. The green channel shows a difference for both controls and the measured sample. This suggests that it may be best to illuminate with a selected wavelength of light that gives the best signal-to-noise ratio for detection of signal from colloidal gold when observing in the vicinity of 580 nm.

In this Example, there are two large changes relative to the device shown and discussed with respect to the Figures. Both of these changes relate to the orientation of the cassette. In this version the cassette is flat relative to the iPhone body and the long axis of the cassette being aligned with the long axis of the iPhone body. The image sensor in the iPhone is asymmetrical with the long axis of the image sensor being aligned with the long axis of the phone body. Orienting the long axis of the detection strip with the long axis of the phone orients the detection strip with the axis of the image sensor that contains the most pixels. The distance between the camera body and the cassette should be the focal length of the lens, in the present configuration 30mm.

The center of the measurement part of the cassette where the sample should be on axis with the center of the camera lens. The center of the light pipe should be in the center of the LED lamp and oriented with its long dimension along the long dimension of the camera. The cut out for the lens and the cut out for the light pipe will leave a fairly thin wall between the two cut outs. Placing a thin wall between the light pipe and the lens prevent the lens from being affected by light coming directly from the illumination source. In addition, it has been observed that the color of the body of the smartphone can affect illumination and the results obtained from an assay. For instance, it was observed that light from a white iPhone flash diffuses more than the light from a black iPhone flash. This confounding factor can, for example, be addressed by an algorithm correction or by placing a gasket or physical barrier around the flash to limit and control light diffusion.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. An enzyme-based assay system for quantification of at least one of the activity level or the concentration of at least one enzyme or an enzyme substrate in a biological sample, the enzyme-based assay system comprising:
a lateral-flow chromatographic assay cassette having an enzymatically activated detectable label configured for assaying a reaction involving an enzyme and a substrate, the lateral-flow chromatographic assay cassette including a sample application zone in fluid communication with a test zone via a fluid transport matrix, wherein the enzymatically activated detectable label is immobilized in the test zone, and wherein the analyte is one of an enzyme or an enzyme substrate;
a testing device for data collection and data analysis, the testing device comprising a handheld computing device selected from the group consisting of a handheld digital camera device, a cellular phone, a smart phone, and a tablet computer, the testing device including:
a testing apparatus configured to be coupled to the handheld computing device and configured to position the lateral-flow chromatographic assay cassette in proximity to a detector and a light source to control for focal length from the detector to the lateral-flow chromatographic assay cassette and and to control for illumination of the lateral-flow chromatographic assay cassette by the light source;
the light source being capable of transmitting at least one wavelength of light configured to yield a detectable signal from the enzymatically activated detectable label; and
the detector comprising a camera positioned to capture the detectable signal from the enzymatically activated detectable label;
at least one focusing lens (e.g., a collimating lens) interposed between the light source, the detector, and the lateral-flow chromatographic assay cassette; and
an interpretive algorithm stored in a computer readable format and electronically coupled to the testing device, wherein the interpretive algorithm is configured to convert the detectable signal from the enzymatically activated detectable label to a numerical value for quantification of at least one of the amount or the activity of at least one enzyme in the sample or the amount of an enzyme substrate in the sample;
the lateral-flow chromatographic assay cassette further includes means for calibrating a response of the enzymatically activated detectable label to a reaction between the enzyme and the substrate, wherein the means for calibration includes at least one of:
a lateral-flow chromatographic assay cassette that includes at least a first calibration standard and a second calibration standard configured to provide at least a two-point calibration curve; or
a lateral-flow chromatographic assay cassette that includes a test strip and a separate calibration strip cassette, wherein the calibration strip includes an enzymatically activated detectable signal configured to provide a known response to a known amount of the enzyme; and
the interpretive algorithm is further configured to (i) calculate a calibration curve and then (ii) convert the detectable signal from the enzymatically activated detectable label to a numerical value for quantification of the amount or the activity of at least one enzyme in the sample.

2. The enzyme-based assay system of claim 1, wherein enzyme is in a mobile phase and the substrate comprises a line of material immobilized in the test zone perpendicular to a flow direction through the fluid transport matrix or the enzymatically activated detectable label is coupled to the substrate and is cleavable in response to enzymatic cleavage of the substrate.

3. The enzyme-based assay system of claim 2, wherein quantification of the amount or the activity of the at least one enzyme in the sample includes a measurement of a loss of the enzymatically activated detectable label from the substrate as a function of time.

4. The enzyme-based assay system of claim 1, wherein the enzymatically activated detectable label is configured to develop a detectable signal in response to enzymatic cleavage of the substrate, and wherein the enzyme and the enzymatically activated detectable label are immobilized to the fluid transport matrix and the substrate is in a mobile phase.

5. The enzyme-based assay system of claim 1, wherein a product of enzymatic cleavage of the substrate interacts with a reporter to yield the enzymatically activated detectable signal.

6. The enzyme-based assay system of claim 1, wherein a product of enzymatic cleavage of the substrate is linked to development of the enzymatically activated detectable signal from a reporter through at least one additional enzymatic reaction.

7. The enzyme-based assay system of claim 6, wherein the at least one additional enzymatic reaction yields a product that interacts with the reporter to yield the enzymatically activated detectable signal.

8. The diagnostic test system of claim 1, wherein the light source is at least one of a camera flash, an autofocus illuminator, ambient light, sunlight, an LED light, an incandescent lamp, or a gas-discharge lamp.

9. The diagnostic test system of claim 1, wherein at least one wavelength filter is interposed between the light source and the lateral-flow chromatographic assay cassette.

10. The diagnostic test system of claim 1, wherein at least one light conducting fiber is interposed between the light source and the lateral-flow chromatographic assay cassette.

11. The diagnostic test system of claim 1, wherein the enzymatically activated detectable label includes at least one of colored beads, colloidal gold, colloidal silver, dyes, fluorescent dyes, an electrochemical detector, a conductivity detector, or quantum dots.

12. The diagnostic test system of claim 1, wherein the detectable signal includes at least one of emission, color intensity, reflectance, diffuse scattering, elastic light scattering, transmission, fluorescence, surface plasmon detection, Rayleigh scattering, electrochemical detection, conductivity, transmission, absorbance, magnetic, or acoustic.

13. A method, comprising:
providing the enzyme-based assay system as defined in any one of claims 1-12;
applying a liquid sample to the lateral-flow chromatographic assay cassette;
inserting the lateral-flow chromatographic assay cassette into the testing apparatus;
illuminating the lateral-flow chromatographic assay cassette to yield a detectable signal from the enzymatically activated detectable label; and
querying an interpretive algorithm stored in a computer readable format and electronically coupled to the testing device, wherein the interpretive algorithm is configured convert the detectable signal from the enzymatically activated detectable label to a numerical value for quantification of at least one of the amount or the activity of at least one enzyme in the sample or the amount of an enzyme substrate in the sample.

14. The method of claim 13, wherein the enzymatically activated detectable label is coupled to the substrate and is cleavable in response to enzymatic cleavage of the substrate, and the method further comprises:
illuminating the lateral-flow chromatographic assay cassette to yield a first detectable signal from the enzymatically activated detectable label;
allowing enzymatic cleavage of the enzymatically activated detectable label from the substrate to proceed for a period of time;
illuminating the lateral-flow chromatographic assay cassette to yield a second detectable signal from the enzymatically activated detectable label, wherein the second detectable signal is reduced relative to the first detectable signal in proportion to the concentration or activity of the enzyme in the liquid sample.

## Patentansprüche

1. Testsystem auf Enzymbasis zum Quantifizieren zumindest der Aktivitätsrate und/oder Konzentration mindestens eines Enzyms oder eines Enzymsubstrats in einer biologischen Probe, wobei das Testsystem auf Enzymbasis umfasst:
eine Testkassette eines chromatographischen Lateral-Flow-Tests mit einem enzymatisch aktivierten detektierbaren Label zum Testen einer Reaktion unter Beteiligung eines Enzyms und eines Substrats, wobei die Testkassette des chromatographischen Lateral-Flow-Tests eine Probenauftragszone aufweist, die über eine Fluidtransportmatrix mit einer Testzone in Fluidverbindung steht, wobei das enzymatisch detektierbare Label in der Testzone immobilisiert wird und wobei der Analyt ein Enzym oder ein Enzymsubstrat ist;
eine Testvorrichtung zur Datengewinnung und Datenanalyse, wobei die Testvorrichtung ein Handheld-Computergerät umfasst, das ausgewählt ist aus der Gruppe, die aus einer Handheld-Digitalkameravorrichtung, einem Mobiltelefon,
einem Smartphone und einem Tablet-Computer besteht, wobei die Testvorrichtung umfasst:
ein Testgerät, das konfiguriert ist zum Verbinden mit dem Handheld-Computergerät und zum Positionieren der Testkassette des chromatographischen Lateral-Flow-Tests in der Nähe eines Detektors und einer Lichtquelle, zum Steuern der Brennweite von dem Detektor zu der Testkassette des chromatographischen Lateral-Flow-Tests und zum Steuern der Beleuchtung der Testkassette des chromatographischen Lateral-Flow-Tests durch die Lichtquelle;
wobei die Lichtquelle geeignet ist zum Senden von Licht mit wenigstens einer Wellenlänge, und wobei das Licht konfiguriert ist zum Hervorbringen eines detektierbaren Signals von dem enzymatisch aktivierten detektierbaren Label; und
wobei der Detektor eine Kamera umfasst, die positioniert ist zum Erfassen des detektierbaren Signals von dem enzymatisch aktivierten detektierbaren Label;
mindestens eine Fokussieroptik (z.B. einen Kollimator), die zwischen die Lichtquelle, den Detektor und die Testkassette des chromatographischen Lateral-Flow-Tests geschaltet ist; und
einen interpretativen Algorithmus, der in einem computerlesbaren Format gespeichert und mit der Testvorrichtung elektronisch verbunden ist, wobei der interpretative Algorithmus konfiguriert ist zum Umwandeln des detektierbaren Signals von dem enzymatisch aktivierten detektierbaren Label in einen numerischen Wert zum Quantifizieren zumindest der Menge und/oder Aktivität des wenigstens einen Enzyms in der Probe oder der Menge eines Enzymsubstrats in der Probe;
wobei die Testkassette des chromatographischen Lateral-Flow-Tests ferner Mittel zum Kalibrieren einer Antwort des enzymatisch aktivierten detektierbaren Labels auf eine Reaktion zwischen dem Enzym und dem Substrat umfasst, wobei das Mittel zum Kalibrieren zumindest enthält:
eine Testkassette des chromatographischen Lateral-Flow-Tests mit mindestens einem ersten Kalibrierstandard und einem zweiten Kalibrierstandard, die konfiguriert sind zum Bereitstellen zumindest einer Zweipunktkalibierungskurve;
und/oder eine Testkassette des chromatographischen Lateral-Flow-Tests mit einem Teststreifen und einer separaten Kalibrierstreifenkassette, wobei der Kalibrierstreifen ein enzymatisch aktiviertes detektierbares Signal enthält, das konfiguriert ist zum Bereitstellen einer bekannten Antwort auf eine bekannte Menge des Enzyms; und wobei der interpretative Algorithmus ferner konfiguriert ist für (i) die Berechnung einer Kalibrierungskurve und (ii) für die anschließende Konvertierung des detektierbaren Signals von dem enzymatisch aktivierten detektierbaren Label in einen numerischen Wert für die Quantifizierung der Menge oder der Aktivität von wenigstens einem Enzym in der Probe.

2. Testsystem auf Enzymbasis nach Anspruch 1, wobei das Enzym in einer mobilen Phase ist und das Substrat eine zu einer Flussrichtung durch die Fluidtransportmatrix senkrechten Linie eines in der Testzone immobilisierten Materials umfasst oder das enzymatisch aktivierte detektierbare Label mit dem Substrat verbunden wird und in Reaktion auf die enzymatische Spaltung des Substrats spaltbar ist.

3. Testsystem auf Enzymbasis nach Anspruch 2, wobei die Quantifizierung der Menge oder der Aktivität des mindestens einen Enzyms in der Probe eine zeitabhängige Messung eines Verlustes des enzymatisch aktivierten detektierbaren Labels aus dem Substrat umfasst.

4. Testsystem auf Enzymbasis nach Anspruch 1, wobei das enzymatisch aktivierte detektierbare Label konfiguriert ist für die Entwicklung eines detektierbaren Signals in Reaktion auf eine enzymatische Spaltung des Substrats und wobei das Enzym und das enzymatisch aktivierte detektierbare Label an der Fluidtransportmatrix immobilisiert sind und das Substrat in einer mobilen Phase ist.

5. Testsystem auf Enzymbasis nach Anspruch 1, wobei ein Produkt einer enzymatischen Spaltung des Substrats mit einem Reporter interagiert, um das enzymatisch aktivierte detektierbare Signal zu liefern.

6. Testsystem auf Enzymbasis nach Anspruch 1, wobei ein Produkt einer enzymatischen Spaltung des Substrats durch mindestens eine zusätzliche enzymatische Reaktion mit einer Entwicklung des enzymatisch aktivierten detektierbaren Signals von einem Reporter verknüpft ist.

7. Testsystem auf Enzymbasis nach Anspruch 6, wobei die mindestens eine zusätzliche enzymatische Reaktion ein Produkt liefert, das mit dem Reporter interagiert, um das enzymatisch aktivierte detektierbare Signal zu liefern.

8. Diagnostisches Testsystem nach Anspruch 1, wobei die Lichtquelle mindestens ein Kamerablitz und/oder eine Autofokusbeleuchtung, ein Umgebungslicht, Sonnenlicht, eine LED-Lampe, eine Glühlampe oder eine Gasentladungslampe ist.

9. Diagnostisches Testsystem nach Anspruch 1, wobei mindestens ein Wellenlängenfilter zwischen die Lichtquelle und die Testkassette des chromatographischen Lateral-Flow-Tests geschaltet ist.

10. Diagnostisches Testsystem nach Anspruch 1, wobei mindestens eine Lichtleitfaser zwischen die Lichtquelle und die Testkassette des chromatographischen Lateral-Flow-Tests geschaltet ist.

11. Diagnostisches Testsystem nach Anspruch 1, wobei das enzymatisch aktivierte detektierbare Label mindestens farbige Perlen und/oder kolloidales Gold, kolloidales Silber, Farbstoffe, fluoreszierende Farbstoffe, einen elektrochemischen Detektor, einen Leitfähigkeitsdetektor oder Quantenpunkte enthält.

12. Diagnostisches Testsystem nach Anspruch 1, wobei das detektierbare Signal mindestens eine Emission und/oder Farbintensität, einen Reflexionswert, eine diffuse Streuung, eine elastische Lichtstreuung, eine Transmission, eine Fluoreszenz, eine Oberflächenplasmondetektion, eine Rayleigh-Streuung, eine elektrochemische Detektion, eine Konduktivität, Transmission, Absorbierung, magnetisch oder akustisch, umfasst.

13. Verfahren, umfassend:
das Bereitstellen eines Testsystems auf Enzymbasis gemäß einem der Ansprüche 1 bis 12;
das Auftragen einer flüssigen Probe auf die Testkassette des chromatographischen Lateral-Flow-Tests;
das Einsetzen der Testkassette des chromatographischen Lateral-Flow-Tests in das Testgerät;
das Beleuchten der Testkassette des chromatographischen Lateral-Flow-Tests zum Hervorbringen eines detektierbaren Signals von dem enzymatisch aktivierten detektierbaren Label; und
das Abfragen eines in einem computerlesbaren Format gespeicherten und in elektronischer Verbindung mit dem Testgerät stehenden interpretativen Algorithmus, wobei der interpretative Algorithmus konfiguriert ist zum Umwandeln des detektierbaren Signals von dem enzymatisch aktivierten detektierbaren Label in einen numerischen Wert zum Quantifizieren mindestens der Menge und/oder der Aktivität des mindestens einen Enzyms in der Probe oder der Menge des Enzymsubstrats in der Probe.

14. Verfahren nach Anspruch 13, wobei das enzymatisch aktivierte detektierbare Label mit dem Substrat verbunden ist und in Reaktion auf die enzymatische Spaltung des Substrats spaltbar ist, wobei das Verfahren ferner umfasst:
das Beleuchten der Testkassette des chromatographischen Lateral-Flow-Tests zum Liefern eines ersten detektierbaren Signals von dem enzymatisch aktivierten detektierbaren Label;
das Ermöglichen des Voranschreitens der enzymatischen Spaltung des enzymatisch aktivierten detektierbaren Labels von dem Substrat für eine Zeitdauer;
das Beleuchten der Testkassette eines chromatographischen Lateral-Flow-Tests zum Liefern eines zweiten detektierbaren Signals von dem enzymatisch aktivierten detektierbaren Label, wobei das zweite detektierbare Signal relativ zu dem ersten detektierbaren Signal im Verhältnis zu der Konzentration oder Aktivität des Enzyms in der flüssigen Probe reduziert ist.

## Revendications

1. Système de dosage enzymatique destiné à permettre de quantifier le niveau d'activité et/ou la concentration d'au moins une enzyme ou d'un substrat enzymatique dans un échantillon biologique, ce système de dosage enzymatique comprenant :
une cassette de dosage chromatographique à flux latéral ayant un marqueur détectable par activation enzymatique conformé pour une réaction impliquant une enzyme et un substrat, la cassette de dosage chromatographique à flux latéral comprenant une zone d'application d'échantillon en communication fluidique avec une zone de test par l'intermédiaire d'une matrice de transport de fluide, le marqueur détectable par activation enzymatique étant immobilisé dans la zone de test, et l'analyte étant une enzyme et/ou un substrat enzymatique,
un dispositif de test permettant de recueillir et d'analyser des données, ce dispositif de test comprenant un dispositif d'ordinateur portatif choisi dans le groupe formé par un dispositif de caméra numérique portatif, un téléphone cellulaire, un Smartphone et une tablette, le dispositif de test comprenant :
un appareil de test conformé pour pouvoir être couplé au dispositif d'ordinateur portatif et pour positionner la cassette de dosage chromatographique à flux latéral à proximité d'un détecteur et d'une source de lumière pour commander la distance focale entre le détecteur et la cassette de dosage chromatographique à flux latéral et pour commander l'éclairage de la cassette de dosage chromatographique à flux latéral par la source de lumière,
la source de lumière étant susceptible de transmettre au moins une longueur d'onde de lumière conformée pour obtenir un signal détectable provenant du marqueur détectable par activation enzymatique,
le détecteur comprenant une caméra positionnée pour saisir le signal détectable provenant du marqueur détectable par activation enzymatique,
au moins une lentille de focalisation (par exemple une lentille collimatrice) interposée entre la source de lumière, le détecteur et la cassette de dosage chromatographique à flux latéral, et
un algorithme interprétatif enregistré sous un format lisible par ordinateur et couplé électroniquement au dispositif de test, l'algorithme interprétatif étant conformé pour transformer le signal détectable provenant du marquage détectable par activation enzymatique en une valeur numérique pour permettre la quantification de la quantité ou de l'activité d'au moins une enzyme dans l'échantillon ou la quantité d'un substrat enzymatique dans l'échantillon,
la cassette de dosage chromatographique à flux latéral comprenant en outre des moyens permettant de calibrer la réponse du marqueur détectable par activation enzymatique à une réaction entre l'enzyme et le substrat, les moyens de calibrage comprenant au moins l'un des éléments suivants :
une cassette de dosage chromatographique à flux latéral comprenant au moins un premier standard de calibrage et un second standard de calibrage conformés pour fournir au moins une courbe de calibrage à deux points, ou
une cassette de dosage chromatographique en flux latéral qui comprend une bandelette de test et une cassette à bandelette de calibrage séparée,
la bandelette de calibrage comprenant un marqueur détectable par activation enzymatique conformé pour fournir une réponse connue à une quantité connue de l'enzyme, et
l'algorithme interprétatif étant en outre conformé pour (i) calculer une courbe de calibrage puis (ii) transformer le signal détectable provenant du marqueur détectable par activation enzymatique en une valeur numérique permettant de quantifier la quantité d'activité d'au moins une enzyme dans l'échantillon.

2. Système de dosage enzymatique conforme à la revendication 1, dans lequel l'enzyme est dans une phase mobile et le substrat comporte une rangée de matériau immobilisé dans la zone de test perpendiculairement à la direction d'écoulement au travers de la matrice de transport de fluide, ou le marqueur détectable par activation enzymatique est couplé au substrat et peut être clivé en réponse au clivage enzymatique du substrat.

3. Système de dosage enzymatique conforme à la revendication 2,
dans lequel la quantification de la quantité ou de l'activité de l'enzyme dans l'échantillon comprend une mesure de la perte du marqueur détectable par activation enzymatique dans le substrat en fonction du temps.

4. Système de dosage enzymatique conforme à la revendication 1,
dans lequel le marqueur détectable par activation enzymatique est conformé pour produire un signal détectable en réponse au clivage enzymatique du substrat, et l'enzyme et le marqueur détectable par activation enzymatique sont immobilisés sur la matrice de transport de fluide et le substrat est dans une phase mobile.

5. Système de dosage enzymatique conforme à la revendication 1,
dans lequel un produit de clivage enzymatique du substrat interagit avec un reporteur pour obtenir le signal détectable par activation enzymatique.

6. Système de dosage enzymatique conforme à la revendication 1,
dans lequel un produit du clivage enzymatique du substrat est lié à la production du signal détectable par activation enzymatique provenant d'un reporteur par au moins une réaction enzymatique additionnelle.

7. Système de dosage enzymatique conforme à la revendication 6,
dans lequel la réaction enzymatique additionnelle donne un produit qui interagit avec le reporteur pour produire le signal détectable par activation enzymatique.

8. Système de test de diagnostic conforme à la revendication 1,
dans lequel la source de lumière est un flash de caméra, et/ou une source lumineuse à mise au point automatique, et/ou la lumière ambiante et/ou la lumière solaire, et/ou la lumière de LED et/ou une lampe à incandescence et/ou une lampe à décharge de gaz.

9. Système de test de diagnostic conforme à la revendication 1,
dans lequel au moins un filtre de longueur d'onde est interposé entre la source de lumière et la cassette de dosage chromatographique à flux latéral.

10. Système de test de diagnostic conforme à la revendication 1,
dans lequel au moins une fibre conductrice de la lumière est interposée entre la source de lumière et la cassette de dosage chromatographique à flux latéral.

11. Système de test de diagnostic conforme à la revendication 1,
dans lequel le marqueur détectable par activation enzymatique comporte des perles colorées et/ou de l'or colloïdal et/ou de l'argent colloïdal et/ou des teintures et/ou des teintures fluorescentes et/ou un détecteur électrochimique et/ou un détecteur de conductivité et/ou des points quantiques.

12. Système de test de diagnostic conforme à la revendication 1,
dans lequel le signal détectable est une émission et/ou une intensité de couleur et/ou une réflexion et/ou une dispersion diffuse et/ou une dispersion de lumière élastique et/ou une transmission et/ou une fluorescence et/ou une détection de plasmon de surface et/ou une dispersion de Rayleigh et/ou une détection électrochimique et/ou une conductivité et/ou une transmission et/ou une absorbance et/ou un signal magnétique et/ou un signal acoustique.

13. Procédé comprenant des étapes consistant à :
se procurer un système de dosage enzymatique conforme à l'une quelconque des revendications 1 à 12,
appliquer un échantillon liquide à la cassette de dosage chromatographique à flux latéral,
introduire la cassette de dosage chromatographique à flux latéral dans l'appareil de test,
éclairer la cassette de dosage chromatographique à flux latéral pour produire un signal détectable provenant du marqueur détectable par activation enzymatique, et
interroger un algorithme interprétatif enregistré sous un format lisible par ordinateur et couplé électroniquement au dispositif de test,
l'algorithme interprétatif étant conformé pour transformer le signal détectable provenant du marqueur détectable par activation enzymatique en une valeur numérique pour permettre de quantifier la quantité et/ou l'activité d'au moins une enzyme dans l'échantillon ou la quantité d'un substrat enzymatique dans l'échantillon.

14. Procédé conforme à la revendication 13,
selon lequel le marqueur détectable par activation enzymatique est couplé au substrat et peut être clivé en réponse au clivage enzymatique du substrat, ce procédé comprenant en outre des étapes consistant à :
éclairer la cassette de dosage chromatographique à flux latéral pour produire un premier signal détectable provenant du marqueur détectable par activation enzymatique,
permettre la mise en oeuvre du clivage enzymatique du marqueur détectable par activation enzymatique dans le substrat pendant un laps de temps,
éclairer la cassette de dosage chromatographique à flux latéral pour produire un second signal détectable provenant du marqueur détectable par activation enzymatique, le second signal détectable étant réduit par rapport au premier signal détectable dans la proportion de la concentration ou de l'activité de l'enzyme dans l'échantillon liquide.
